(19) Europäisches Patentamt
European Patent Office
Office européen des brevets

(11) Publication number: 0 168 965
B1

(12) **EUROPEAN PATENT SPECIFICATION**

(45) Date of publication of patent specification: **09.11.88**

(51) Int. Cl.[4]: **C 07 D 233/64,** A 61 K 31/415

(21) Application number: **85304239.8**

(22) Date of filing: **14.06.85**

(54) **4(5)-substituted imidazoles.**

(30) Priority: **18.06.84 US 621597**

(43) Date of publication of application:
**22.01.86 Bulletin 86/04**

(45) Publication of the grant of the patent:
**09.11.88 Bulletin 88/45**

(84) Designated Contracting States:
**AT BE CH DE FR GB IT LI NL SE**

(56) References cited:
**EP-A-0 072 615**
**US-A-2 946 804**

(73) Proprietor: **ELI LILLY AND COMPANY**
**Lilly Corporate Center**
**Indianapolis Indiana 46285 (US)**

(72) Inventor: **Hirsch, Kenneth Steven**
**R.R. 1 Box BC23**
**New Palestine Indiana 46163 (US)**
Inventor: **Taylor, Harold Mellon**
**7459 Steinmeier Drive**
**Indianapolis Indiana 46250 (US)**

(74) Representative: **Tapping, Kenneth George et al**
**Erl Wood Manor**
**Windlesham Surrey, GU20 6PH (GB)**

**EP 0 168 965 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention provides imidazole derivatives of the formula I

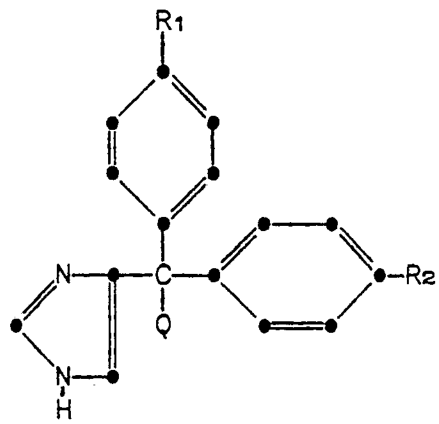

(I)

wherein

Q is hydrogen or hydroxy and

$R_1$ and $R_2$ are independently fluoro or chloro, and pharmaceutically acceptable salts thereof.

By virtue of their ability to inhibit the enzyme aromatase, the compounds of the above formula are useful in the treatment and prevention of estrogen-dependent diseases, especially breast cancer, in mammals.

Estrogens are synthesized from androgenic steroids. In the biosynthetic pathway for estrogen formation, aromatization is an essential step. It is generally believed that if the aromatase enzyme could be effectively inhibited, a useful treatment for estrogen dependent disorders could be obtained [see *Cancer Research,* Vol. 42, Suppl. 8:3261s (1982)].

Several estrogen dependent diseases exist which could be treated with aromatase inhibitors. These include breast cancer, endometriosis, polycystic ovarian disease, benign breast disease, and endometrial cancer. A beneficial effect of antiestrogens in the treatment of breast cancer has been well established [see *Br. J. Cancer, 25,* 270 (1971)]. Two of the known aromatase inhibitors, testolactone and aminoglutethimide, have shown a beneficial effect in treatment of breast cancer. See *Cancer Research, supra.*

Endometriosis is characterized by an abnormal proliferation of the endometrium of the uterus. Since the endometrium is dependent on estradiol for its growth, an inhibitor of estrogen production should stop the progression of the disease.

Benign breast disease, or often called fibrocystic breast disease, appears to be dependent on ovarian steroids. See *Cancer, 49,* 2534 (1982). Aromatase inhibitors have not been tried in this disease, but antiestrogens seem to be of benefit. See *Obstet. Gynecol., 54,* 80 (1979).

Polycystic ovarian disease is one of the most common causes of infertility in women. The disease appears to result from an abnormality in steroid metabolism, and the major form of therapy in this disease is the antiestrogen, clomiphene. See *Clin. Endocrinol., 12,* 177 (1980).

The invention also provides pharmaceutical formulations comprising one or more of the compounds of the above formula in combination with a suitable pharmaceutical carrier, diluent, or excipient therefor. The formulations provided by this invention are particularly useful in treating mammals suffering from estrogen-dependent diseases such as breast cancer.

As will be recognized by those skilled in the art, many of the compounds used in this invention contain an asymmetric carbon atom. This invention is not limited to any particular isomer but includes the individual enantiomers as well as the racemates of the compounds.

Furthermore, it will be recognized that the compounds of the above formula, which are drawn as 4-substituted imidazoles, exist in equilibrium with the corresponding 5-substituted imidazole tautomers, and that reference to the compounds of this invention embodies both of these tautomers. The compounds are therefore referred to as 4(5)-substituted imidazoles.

The pharmaceutically acceptable acid addition salts used in this invention include salts derived from inorganic acids such as hydrochloric acid, nitric acid, phosphoric acid, sulfuric acid, hydrobromic acid, hydriodic acid or phosphorous acid, as well as salts derived from organic acids such as aliphatic mono- and di-carboxylic acids, phenyl-substituted alkanoic acids, hydroxy-alkanoic and -alkanedioic acids, aromatic acids, aliphatic or aromatic sulfonic acids. Typical pharmaceutically acceptable salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate,

phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, malate, tartrate, methanesulfonate, propanesulfonate, naphthalene-1-sulfonate or naphthalene-2-sulfonate. The preferred salts of this invention are those derived from inorganic acids, especially hydrochloric acid.

The compounds of this invention can be prepared by methods described in the art. The compounds employed in this invention are generically taught in British Patent Application GB 2,101,114; however, none of the presently claimed compounds are specifically disclosed. The presently claimed compounds can be prepared by the methods described in the British Application. In addition, following the general procedure for making 2-substituted imidazoles as taught in U.S. Patent No. 4,152,441, the following schemes summarize general methods to prepare the compounds of formula I wherein Q is hydrogen or hydroxy:

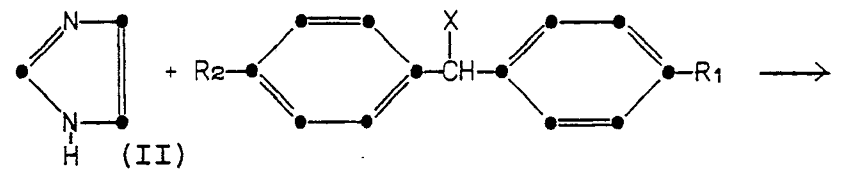

(II) I (Q = H)

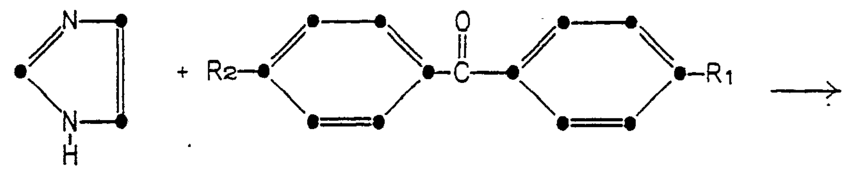

(II) I (Q = OH)

wherein X is chloro or bromo. According to the above schemes, imidazole (II) is treated with a strong alkali metal base, such as sodium hydride or n-butyllithium, in a non-reactive solvent, such as dimethylformamide or tetrahydrofuran. This reaction provides a mixture of the 1-, 2- and 4-alkali metal derivatives of imidazole which is then reacted with the corresponding methyl halide or ketone derivative. The reaction is generally complete within 2—24 hours when the reaction is allowed to proceed at temperatures from about 0—100°C. The reaction provides the corresponding derivatives of formula I wherein Q is hydrogen or hydroxy, together with the undesirable 1- and 2-substituted imidazoles. The isomeric materials may be isolated by known procedures, such as chromatography or crystallization.

An alternate procedure for preparing the carbinol derivatives of formula I is summarized by the following scheme:

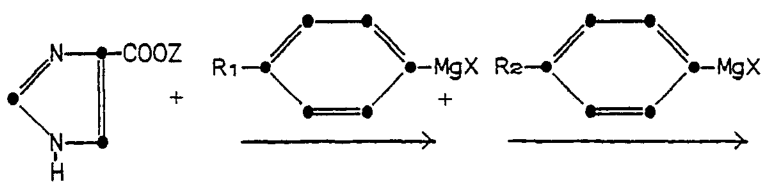

I (Q = OH)

wherein Z is, for instance, $C_1$—$C_4$ alkyl, and X is chloro or bromo. In this procedure, an ester of 4-imidazole carboxylic acid is treated with the appropriate Grignard reagents following standard procedures. The reaction is generally carried out in a mutually compatible solvent, such as an ether, at temperatures from about −20 to 60°C. This process is taught by Zaugg et al. in *J. Org. Chem., 23,* 847 (1958).

The carbinol derivatives (Q is hydroxy) can also be prepared from the hydrogen compound by treating a basic solution of the hydrogen compound with air or oxygen. Conversely, the carbinol derivative may be transformed to the hydrogen compound following the procedure of U.S. Patent No. 2,727,895.

Accordingly, the invention provides a process for preparing a compound of the formula I

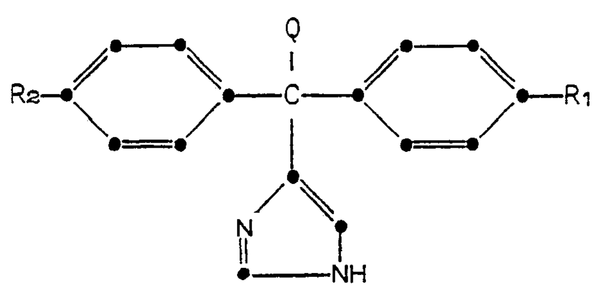

(I)

wherein

Q is hydrogen or hydroxy and

$R_1$ and $R_2$ are independently F or Cl,

or a pharmaceutically acceptable salt thereof, comprising

(a) reacting a compound of formula II

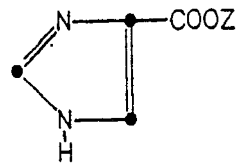

(II)

first with a Grignard reagent of formula

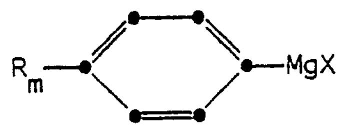

and then with a Grignard reagent of the formula

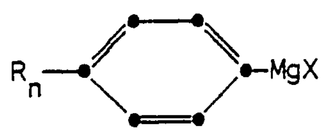

where Z is $C_1$—$C_4$ alkyl, one of $R_m$ and $R_n$ is $R_1$ and the other is $R_2$, and X is chloro or bromo, to produce a compound of formula I wherein Q is hydroxy, or

(b) reacting imidazole in sequence with a strong alkali metal base and a compound of the formula

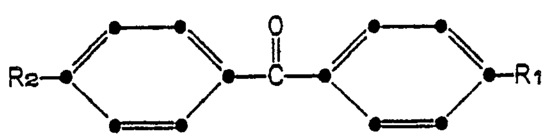

and separating the isomers obtained to produce a compound of formula I wherein Q is hydroxy, or

(c) reacting imidazole in sequence with a strong alkali metal base and a compound of the formula

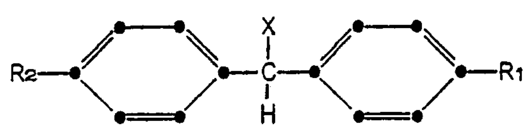

where X is chloro or bromo, and separating the isomers obtained to produce a compound of formula I wherein Q is hydrogen; and

(d) optionally salifying the product of formula I.

In order to more fully illustrate the preparation of the compounds of this invention, the following examples are provided.

Example 1

4(5)-[Bis(4-fluorophenyl)methyl]-1H-imidazole

Nine and two-tenths grams of sodium hydride (57% in oil) were added to a solution of 22 g of imidazole in 200 ml of dimethylformamide with stirring. When the reaction was complete as evidenced by cessation of foaming, 22 g of 4,4′-difluorodiphenylmethyl chloride were added with stirring. The mixture was stirred for 2 hours at room temperature and then warmed on a steam bath for 1.5 hours. The mixture was poured into an ice-water mixture, ether was added, and the layers were separated. The aqueous layer was washed with ether, and the combined ether extracts were washed with water, dried over magnesium sulfate, filtered, and evaporated. The residue was chromatographed over silica gel eluting successively with 10% ethyl acetate in toluene, 20% ethyl acetate in toluene, and 1:1 ethyl acetate/toluene (2 liters each mixture) to provide 1.2 g of 2-[bis(4-fluorophenyl)methyl]imidazole ($R_f$ = 0.44, silica gel TLC plates eluting with 1:1 ethyl acetate/toluene), 14.5 g of 1-[bis(4-fluorophenyl)methyl]imidazole ($R_f$ = 0.30), and 1.99 g of the

desired 4(5)-[bis(4-fluorophenyl)methyl]imidazole ($R_f$ = 0.15). Structures were confirmed by NMR and infrared spectroscopy.

Example 2

4(5)-[(4-Chlorophenyl)(4-fluorophenyl)methyl]imidazole

Following the general procedure of Example 1, the title compound was prepared from imidazole and 4-chlorophenyl-4-fluorophenylmethyl chloride in 3.1% yield.

Example 3

α,α-bis(4-chlorophenyl)-4(5)-imidazolemethanol

A Grignard reagent was prepared from 4.7 g of magnesium turnings, a catalytic amount (four drops) of 1,2-dibromomethane in 2 ml of diethyl ether, and 25.0 g of 4-bromochlorobenzene in 100 ml of tetrahydrofuran. After stirring for approximately two hours, 5.0 g of methyl 4-imidazolecarboxylate were added as a solution in 50 ml of tetrahydrofuran. The mixture was heated at reflux for one hour. The tetrahydrofuran was removed by evaporation and the remaining mixture was poured into an iced ammonium chloride solution and extracted with ethyl acetate. Evaporation of the extract provided a yellow oil which was purified by chromatograph over silica gel eluting with a gradient of ethyl acetate/15% methanol in ethyl acetate. The appropriate fractions were combined, evaporated, and crystallized from ethyl ether/hexane to provide 6.47 g of the title product. One gram of material was recrystallized from benzene to provide 540 mg of product with a melting point of 110—113°C.

Analysis for $C_{16}H_{12}Cl_2N_2O$:

| | | | | |
|---|---|---|---|---|
| Calculated: | C, 60.41; | H, 3.79; | N, 8.78; | Cl, 22.21; |
| Found: | C, 59.99; | H, 4.03; | N, 8.54; | Cl, 22.29. |

The compounds of this invention are useful in preventing or therapeutically treating estrogen-dependent diseases, including breast cancer, in mammals by virtue of their ability to inhibit the enzyme aromatase. Their ability to inhibit aromatase was demonstrated by employing a modification of the isolated rat ovarian microsome method of Brodie et al. in *J. Steroid Biochem., 7,* 787 (1976). In this test system, ovarian microsomes are obtained from rats treated with pregnant mares serum gonadotropin. Test compounds are added to reaction vials containing 0.1 μM 4-androstene-3,17-dione, 100,000 dpm 1,2[$^3$H]-androstenedione, the microsomes and a NADPH generating system. The concentrations of the inhibitors tested ranged between 0.005 and 10 μm. In this assay, aromatization of androstenedione results in the production of [$^3$H]-$H_2O$ which is isolated by extracting the samples with chloroform and treating the aqueous phase with charcoal to remove the free steroid. Samples are counted in a liquid scintillation spectrometer and the percent inhibition determined by comparing the results with control samples incubated without inhibitor. Potency is determined based on the concentration of inhibitor in μM required to produce a 50% inhibition of enzyme activity ($EC_{50}$) when the concentration of substrate (androstenedione) is 0.1 μM. The $EC_{50}$'s of certain of the compounds of the above formula are summarized in Table 1.

Table 1

Aromatase Inhibition in the Rat Ovarian Microsome Assay

| Compound of Example | $EC_{50}$* |
|---|---|
| 1 | <0.05 |
| 2 | <0.05 |
| 3 | <0.05 |

*Concentration of compound in μM required to achieve 50% inhibition of aromatase activity when substrate concentration is 0.1 μM.

By virtue of their ability to inhibit the enzyme aromatase, the compounds of this invention are able to inhibit the synthesis of estrogens in mammals, thereby making the compounds useful in the treatment of estrogen-dependent diseases, such as breast cancer. This activity was demonstrated in the following *in vivo* test system.

Estrogen Synthesis Inhibition in Rats

Immature female Wistar rats (45—55 grams) were divided into control and test groups of 4—5 animals each. Test compounds were administered for seven days daily by gavage in corn oil. Control animals received corn oil without the test compound. Beginning on the fourth day of the test, all animals treated with the test compound and one half of the control animals were given a subcutaneous injection of 1.0 mg of testosterone propionate in corn oil. The remaining control animals received only an equivalent volume of corn oil. On the seventh day of the test, rats treated with testosterone propionate were injected subcutaneously with 100 µCi of [$^3$H]-testosterone in 50 µl of 3:1 (v/v) saline-ethanol.

After two hours, the animals were killed by decapitation. Uteri were isolated, trimmed of extraneous connective tissue, and weighed. As summarized in Table 2 below, the corn oil treated animals exhibited low uterine weight and represent unstimulated or negative controls. In the control animals treated with testosterone propionate, estrogens produced by aromatization stimulated the uterus resulting in an increase in weight. Compounds which inhibit aromatization produced urerine weights significantly lower than those of the testosterone treated controls.

Ovaries from rats treated with [$^3$H]-testosterone were excised, cleaned of extraneous tissue, and homogenized in 2.5 ml of a 1.0 mM potassium phosphate buffer containing 3.0 mM $MgCl_2 \cdot 6H_2O$, 320 mM sucrose, and 0.25% Triton X-100 (polyethylene glycol p-isooctyl phenyl ether, Rohm and Haas) at pH 6.5. The ovarian steroids were extracted with 1.5 ml of 9:1 (v/v) toluene/ethanol to which had been added 25 to 100 mcg each of unlabelled estradiol, estriol, and estrone, and approximately 1000 dpm of [$^{14}$C]-estradiol. The samples were vortexed, centrifuged at 500 × g for 10 minutes, and the organic phase was transferred to a conical vial. Two additional extractions were performed on the residue in the same way. The pooled organic extracts were evaporated for subsequent thin-layer chromatography.

Ovarian proteins were precipitated by the addition of 5.0 ml of ethanol to the remaining aqueous phase. After an overnight incubation at 4°C, the samples were centrifuged at 1500 × g for 10 minutes. The supernatant was discarded and the pellet was dissolved in 0.3 N potassium hydroxide. Protein was determined according to the method of Bradford, *Analytical Biochemistry, 72,* 248 (1976).

The organic residue from each above extraction was redissolved in 9:1 (v/v) dichloromethane/methanol. The solution of each sample was applied to separate silica gel thin layer chromatography plates which contained a fluorescent indicator. The plates were developed in the first dimension with 160:38:1.5:0.5 (v/v/v/v) dichloromethane/ethyl acetate/methanol/acetic acid to within 3 cm of the top of the plate. After air-drying, the plate was developed in the second dimension with 180:19:1 (v/v/v) dichloromethane/methanol/ammonium hydroxide. The plate was air-dried and viewed under 254 nm UV light.

The visible spots were marked and the plates were sprayed with primulin (0.001% in 4:1 v/v acetone/water) according to the method of Wright, *J. Chromatography, 59,* 220 (1971) which allowed for the identification of additional steroids under 365 nm UV light. The spots were scraped from the plate using a glass wool plugged Pasteur pipet attached to a vacuum line. The steroids were eluted directly into scintillation vials by the addition of 0.2 ml of dichloromethane followed by two washes each of 2.0 ml of methanol. The organic solvent was evaporated and 10.0 ml of scintillation fluid (Beckman Ready Solv-NA) was added to the vials. Samples were analyzed by liquid scintillation spectrometry. Corrections were made based on the recoveries of the [$^{14}$C]-steroid. Steroid concentrations are expressed as femtomoles per milligram protein.

Table 2

Effects of a Compound of Formula I on estrogen levels and uterine weight

| Test No. | Compound | Dose* | Animals | Mean Uterine Weight (mg) | Mean Steroid Concentration** estradiol | estrone | estriol |
|---|---|---|---|---|---|---|---|
| I | α,α-bis(4-chlorophenyl)-4(5)-imidazolemethanol | 1 | 5 | 214.2 | 0.35 | 0.00 | 0.132 |
| | | 3 | 5 | 158.8+ | 0.31 | 0.05 | 0.383 |
| | | 10 | 5 | 131.2+ | 0.19+ | 0.03 | 0.218 |
| | | 30 | 5 | 96.2+ | 0.22 | 0.07 | 0.391 |
| | | 100 | 4 | 79.5+ | 0.10+ | 0.36+ | 0.534+ |
| | Testosterone-treated control | - | 5 | 217.8 | 0.40 | 0.07 | 0.165 |
| | Corn oil control | - | 5 | 93.0+ | - | - | - |

* mg/kg p.o. by gavage

** femtomoles per milligram of protein.

\+ significantly different from testosterone-treated control, $p<0.05$.

The compounds of this invention may be administered by any number of routes, including the oral, subcutaneous, intramuscular, intravenous, transdermal, and rectal routes. The compounds are usually employed in the form of pharmaceutical compositions. Such compositions are prepared in a manner well known in the pharmaceutical art and comprise from about 1 to about 95 percent by weight of at least one active compound of the formula I.

Such pharmaceutical compositions comprise as active ingredient a compound of the above formula associated with a pharmaceutically acceptable carrier. In making the compositions, the active ingredient will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the composition can be in the form of tablets, pills, powders, lozenges, sachets, cachets, elixirs, emulsions, solutions, syrups, suspensions, aerosols (as a solid or in a liquid medium), ointments containing for example up to 10% by weight of the active compound, soft and hard gelatin capsules, suppositories, sterile injectable solutions, and sterile packaged powders.

Some examples of suitable carriers, excipients, and diluents include lactose, dextrose, sucrose, sorbitol, mannitol, starches, gum acacia, calcium phosphate, alginates, calcium silicate, microcrystalline cellulose, polyvinylpyrrolidone, cellulose, tragacanth, gelatin, syrup, methyl cellulose, methyl- and propylhydroxybenzoates, talc, magnesium stearate, water, and mineral oil. The formulations can additionally include lubricating agents, wetting agents, emulsifying and suspending agents, preserving agents, sweetening agents or flavoring agents. The compositions may be formulated so as to provide quick, sustained, or delayed release of the active ingredient after administration to the patient by employing procedures well known in the art.

For oral administration, a compound of this invention can be admixed with carriers and diluents molded into tablets or enclosed in gelatin capsules. The mixtures can alternatively be dissolved in liquids such as ten percent aqueous glucose solution, isotonic saline or sterile water, and administered intravenously or by injection. Such solutions can, if desired, be lyophilized and stored in a sterile ampoule ready for reconstitution by the addition of sterile water for ready intramuscular injection.

The compositions are preferably formulated in a unit dosage form, each dosage containing from about 1 to about 500 mg, more usually about 5 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with the required pharmaceutical carrier.

The active compounds are effective over a wide dosage range. For example, dosages per day will normally fall within the range of about 0.05 to about 300 mg/kg of body weight. In the treatment of adult humans, the range of about 0.1 to about 50 mg/kg, in single or divided doses, is preferred. However, it will be understood that the amount of the compound actually administered will be determined by a physician, in the light of the relevant circumstances including the condition to be treated, the choice of compound to be administered, the age, weight, and response of the individual patient, the severity of the patient's symptoms, and the chosen route of administration, and therefore the above dosage ranges are not intended to limit the scope of the invention in any way.

In order to more fully illustrate the operation of this invention, the following formulation examples are provided. The examples are illustrative only and are not intended to limit the scope of the invention. The formulations employ as active compounds any of the pharmaceutical compounds of the above formula.

### Example 4

Hard gelatin capsules are prepared using the following ingredients:

| | per capsule |
|---|---|
| 4(5)-[(4-fluorophenyl)(4-chlorophenyl)methyl]-imidazole | 250 mg |
| Starch dried | 200 mg |
| Magnesium stearate | 10 mg |
| Total | 460 mg |

The above ingredients are mixed and filled into hard gelatin capsules in 460 mg quantities.

Example 5

Capsules each containing 20 mg of active ingredient are made as follows:

| | per capsule |
|---|---|
| 4(5)-[(4-fluorophenyl)(4-chlorophenyl)methyl]-imidazole | 20 mg |
| Starch | 89 mg |
| Microcrystalline cellulose | 89 mg |
| Magnesium stearate | 2 mg |
| Total | 200 mg |

The active ingredient, cellulose, starch and magnesium stearate are blended, passed through a No. 45 mesh U.S. sieve and filled into hard gelatin capsules in 200 mg quantities.

Example 6

Capsules each containing 100 mg of active ingredient are made as follows:

| | per capsule |
|---|---|
| α,α-bis(4-chlorophenyl)-4(5)-imidazolemethanol | 100 mg |
| Polyoxyethylenesorbitan monooleate | 50 mcg |
| Starch powder | 250 mg |

The above ingredients are thoroughly mixed and are placed in an empty gelatin capsule.

Example 7

Tablets each containing 10 mg of active ingredient are made up as follows:

| | per tablet |
|---|---|
| 4(5)-[1,1-bis(4-fluorophenyl)methyl]-imidazole | 10 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 100 mg |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50—60°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 100 mg.

Example 8

A tablet formula is prepared using the ingredients below:

| | per tablet |
|---|---|
| 4(5)-[(4-fluorophenyl)(4-chlorophenyl)-methyl]imidazole | 250 mg |
| Cellulose microcrystalline | 400 mg |
| Silicon dioxide fumed | 10 mg |
| Stearic acid | 5 mg |
| Total | 665 mg |

The components are blended and compressed to form tablets each weighing 665 mg.

Example 9

Suppositories each containing 25 mg of active ingredient are made as follows:

| | per suppository |
|---|---|
| α-(4-fluorophenyl)-α-(4-chlorophenyl)-4(5)-imidazolemethanol | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

Example 10

Suspensions each containing 5 mg of active ingredient per 5 ml dose are made as follows:

| | per 5 ml of suspension |
|---|---|
| 4(5)-[bis(4-fluorophenyl)methyl]imidazole | 5 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to | 5 ml |

The active ingredient is passed through a No. 45 mesh U.S. sieve and mixed with the sodium carboxymethylcellulose and syrup to form a smooth paste. The benzoic acid solution, flavor and color is diluted with some of the water and added, with stirring. Sufficient water is then added to produce the required volume.

Example 11

An aerosol solution is prepared containing the following components:

| | Weight % |
|---|---|
| α-(4-fluorophenyl)-α-(4-chlorophenyl)-4(5)-imidazolemethanol | 0.25 |
| Ethanol | 29.75 |
| Propellant 22 (Chlorodifluoromethane) | 70.00 |

The active compound is mixed with ethanol and the mixture added to a portion of the propellant 22, cooled to −30°C and transferred to a filling device. The required amount is then fed to a stainless steel container and diluted further with the remaining amount of propellant. The valve units are then fitted to the container.

**Claims for the Contracting States: BE CH DE FR GB IT LI LU NL SE**

1. A compound of the formula I

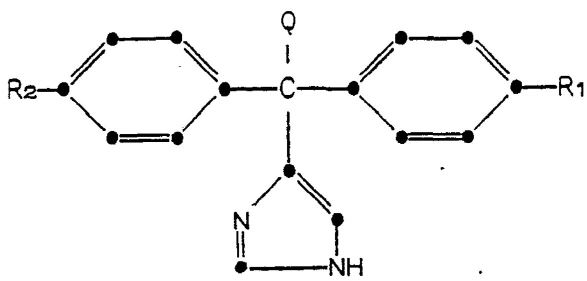

(I)

wherein

Q is hydrogen or hydroxy and

$R_1$ and $R_2$ are independently F or Cl,

or a pharmaceutically acceptable salt thereof.

2. α,α-Bis(4-chlorophenyl)-1H-imidazole-4(5)-methanol, or a pharmaceutically acceptable salt thereof.

3. 4-[(4-Chlorophenyl)(4-fluorophenyl)methyl]-1H-imidazole, or a pharmaceutically acceptable salt thereof.

4. 4-Bis(4-fluorophenyl)methyl]-1H-imidazole, or a pharmaceutically acceptable salt thereof.

5. Use of a compound of any one of claims 1 to 4 in preparation of a medicament for inhibiting aromatase or for treating or preventing estrogen dependent disease.

6. A pharmaceutical formulation comprising a compound of any one of claims 1 to 4 as active ingredient in association with one or more pharmaceutically acceptable excipients or carriers therefor.

7. A process for preparing a compound of the formula I

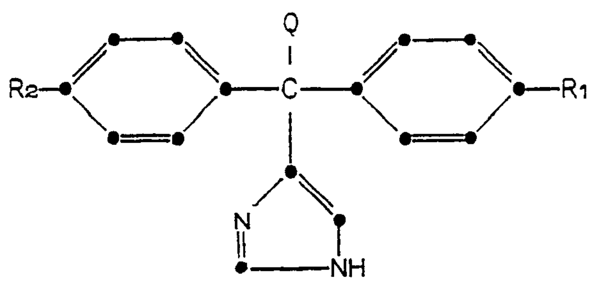

(I)

wherein

Q is hydrogen or hydroxy and

$R_1$ and $R_2$ are independently F or Cl,

or a pharmaceutically acceptable salt thereof, comprising

(a) reacting a compound of formula II

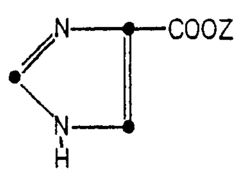

(II)

first with a Grignard reagent of formula

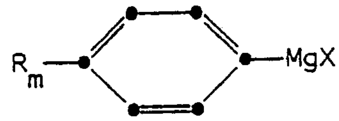

and then with a Grignard reagent of the formula

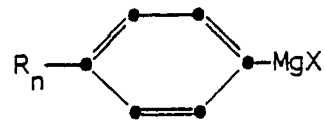

where Z is $C_1$—$C_4$ alkyl, one of $R_m$ and $R_n$ is $R_1$ and the other is $R_2$, and X is chloro or bromo, to produce a compound of formula I wherein Q is hydroxy, or

(b) reacting imidazole in sequence with a strong alkali metal base and a compound of the formula

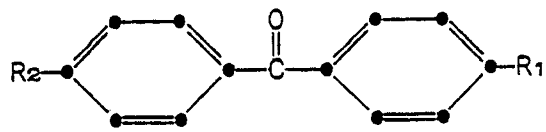

and separating the isomers obtained to produce a compound of formula I wherein Q is hydroxy, or

(c) reacting imidazole in sequence with a strong alkali metal base and a compound of the formula

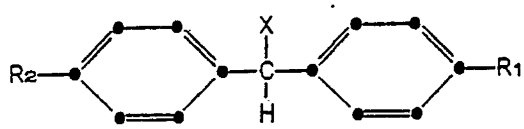

where X is chloro or bromo, and separating the isomers obtained to produce a compound of formula I wherein Q is hydrogen; and

(d) optionally salifying the product of formula I.

**Claims for the Contracting State: AT**

1. A process for preparing a compound of the formula I

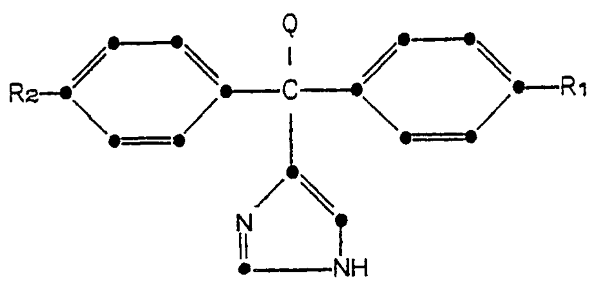

(I)

wherein

Q is hydrogen or hydroxy and

$R_1$ and $R_2$ are independently F or Cl,

or a pharmaceutically acceptable salt thereof, comprising

(a) reacting a compound of formula II

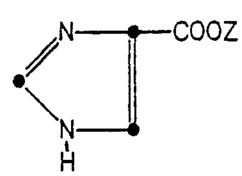

(II)

first with a Grignard reagent of formula

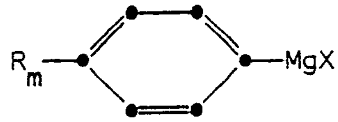

and then with a Grignard reagent of the formula

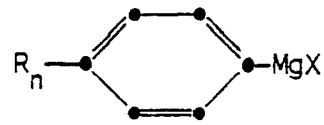

where Z is $C_1$—$C_4$ alkyl, one of $R_m$ and $R_n$ is $R_1$ and the other is $R_2$, and X is chloro or bromo, to produce a compound of formula I wherein Q is hydroxy, or

(b) reacting imidazole in sequence with a strong alkali metal base and a compound of the formula

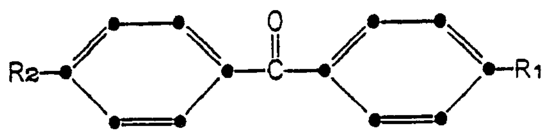

and separating the isomers obtained to produce a compound of formula I wherein Q is hydroxy, or

(c) reacting imidazole in sequence with a strong alkali metal base and a compound of the formula

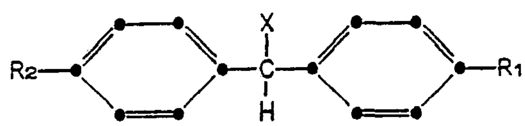

where X is chloro or bromo, and separating the isomers obtained to produce a compound of formula I wherein Q is hydrogen; and

(d) optionally salifying the product of formula I.

2. The process of claim 1 wherein a compound of formula

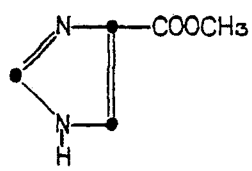

is reacted with at least two equivalents of a Grignard reagent of the formula

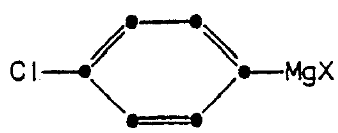

to produce α,α-Bis(4-chlorophenyl)-1H-imidazole-4(5)-methanol, and the product is optionally salified.

3. The process of claim 1 wherein imidazole is reacted in sequence with a strong alkali metal base and a compound of the formula

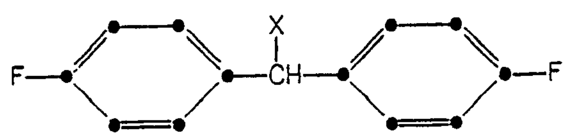

wherein X is chloro or bromo, to produce 4(5)-[Bis(4-fluorophenyl)methyl]-1H-imidazole, and the product is optionally salified.

4. The process of claim 1 wherein imidazole is reacted in sequence with a strong alkali metal base and a compound of the formula

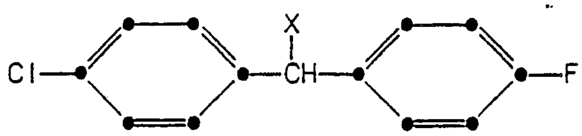

wherein X is chloro or bromo, to produce 4(5)-[(4-chlorophenyl)(4-fluorophenyl)methyl]-imidazole, and the product is optionally salified.

**Patentansprüche für die Vertragsstaaten: BE CH LI DE FR GB IT LU NL SE**

1. Verbindung der Formel (I)

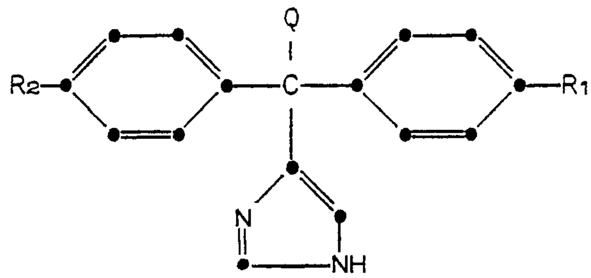

(I)

worin

Q Wasserstoff oder Hydroxy ist und

$R_1$ sowie $R_2$ unabhängig Fluor oder Chlor bedeuten,

oder ein pharmazeutisch annehmbares Salz hiervon.

2. α,α-Bis(4-Chlorphenyl)-1H-imidazol-4(5)-methanol oder ein pharmazeutisch annehmbares Salz hiervon.

3. 4-[(4-Chlorphenyl)(fluorphenyl)methyl]-1H-imidazol oder ein pharmazeutisch annehmbares Salz hiervon.

4. 4-[Bis(4-fluorphenyl)methyl]-1H-imidazol oder ein pharmazeutisch annehmbares Salz hiervon.

5. Verwendung einer Verbindung nach irgend einem der Ansprüche 1 bis 4 zur Herstellung eines Arzneimittels für die Hemmung von Aromatase oder für die Behandlung oder Verhinderung oestrogenabhängiger Krankheitszustände.

6. Pharmazeutische Formulierung, dadurch gekennzeichnet, daß sie eine Verbindung nach irgend einem der Ansprüche 1 bis 4 als Wirkstoff in Verbindung mit einem oder mehreren pharmazeutisch annehmbaren Hilfsstoffen oder Trägern hierfür enthält.

7. Verfahren zur Herstellung einer Verbindung der Formel (I)

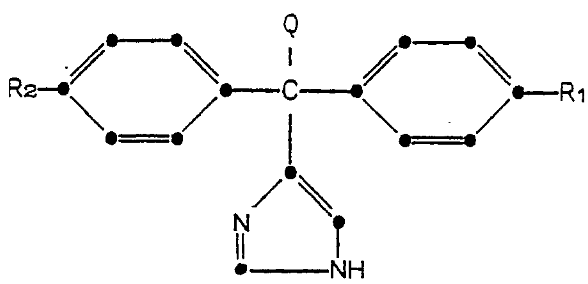

(I)

worin

Q Wasserstoff oder Hydroxy ist und

$R_1$ sowie $R_2$ unabhängig Fluor oder Chlor bedeuten,

oder eines pharmazeutisch annehmbaren Salzes hiervon, dadurch gekennzeichnet, daß

(a) eine Verbindung der Formel (II)

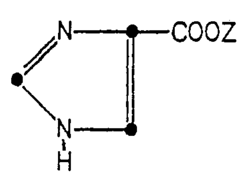

(II)

zuerst mit einem Grignard-Reagens der Formel

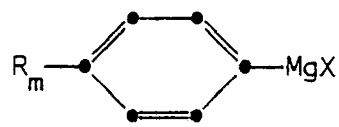

und dann mit einem Grignard-Reagens der Formel

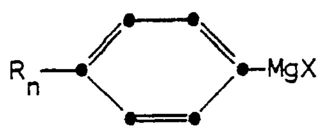

worin Z für $C_1$—$C_4$-Alkyl steht, einer der Substituenten $R_m$ und $R_n$ dem Substituenten $R_1$ und der andere dem Substituenten $R_2$ entspricht und X Chlor oder Brom ist, umgesetzt und so eine Verbindung der Formel (I) gebildet wird, worin Q Hydroxy ist oder

(b) Imidazol der Reihe nach mit einer starken Alkalimetallbase und einer Verbindung der Formel

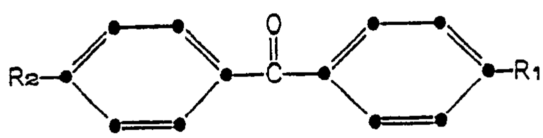

umgesetzt wird und die erhaltenen Isomeren aufgetrennt werden und so eine Verbindung der Formel (I) gebildet wird, worin Q Hydroxy ist, oder

(c) Imidazol der Reihe nach mit einer starken Alkalimetallbase und einer Verbindung der Formel

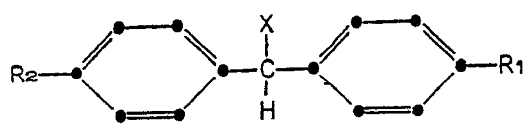

worin X Chlor oder Brom ist, umgesetzt wird und die erhaltenen Isomeren aufgetrennt werden und so eine Verbindung der Formel (I) gebildet wird, worin Q Wasserstoff ist, und

(d) das Produkt der Formel (I) gegebenenfalls in-ein Salz überführt wird.

**Patentansprüche für den Vertragsstaat: AT**

1. Verfahren zur Herstellung einer Verbindung der Formel (I)

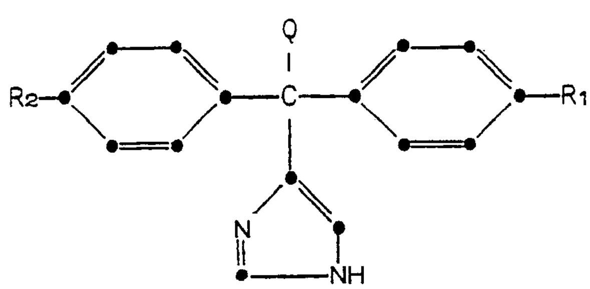

(I)

worin

Q Wasserstoff oder Hydroxy ist und

$R_1$ sowie $R_2$ unabhängig Fluor oder Chlor bedeuten,

oder eines pharmazeutisch annehmbaren Salzes hiervon, dadurch gekennzeichnet, daß

(a) eine Verbindung der Formel (II)

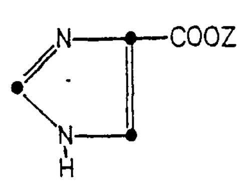

(II)

zuerst mit einem Grignard-Reagens der Formel

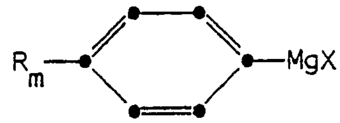

und dann mit einem Grignard-Reagens der Formel

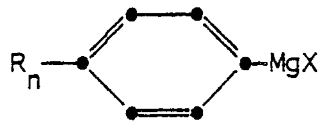

worin Z für $C_1$—$C_4$-Alkyl steht, einer der Substituenten $R_m$ und $R_n$ dem Substituenten $R_1$ und der andere dem Substituenten $R_2$ entspricht und X Chlor oder Brom ist, umgesetzt und so eine Verbindung der Formel (I) gebildet wird, worin Q Wasserstoff ist oder

(b) Imidazol der Reihe nach mit einer starken Alkalimetallbase und einer Verbindung der Formel

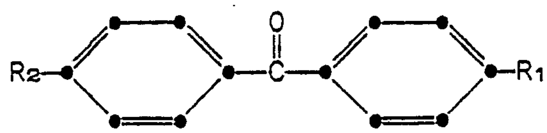

umgesetzt wird und die erhaltenen Isomeren aufgetrennt werden und so eine Verbindung der Formel (I) gebildet wird, worin Q Hydroxy ist, oder

(c) Imidazol der Reihe nach mit einer starken Alkalimetallbase und einer Verbindung der Formel

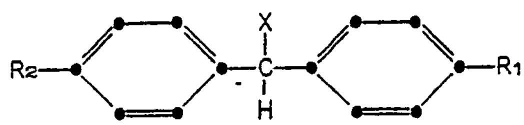

worin X Chlor oder Brom ist, umgesetzt wird und die erhaltenen Isomeren aufgetrennt werden und so eine Verbindung der Formel (I) gebildet wird, worin Q Wasserstoff ist, und

(d) das Produkt der Formel (I) gegebenenfalls in ein Salz überführt wird.

2. Verfahren nach Anspruch 1, dadurch gekennzeichnet, daß eine Verbindung der Formel

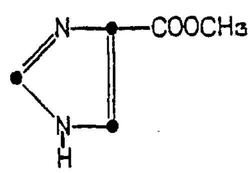

mit wenigstens zwei Äquivalenten eines Grignard-Reagens der Formel

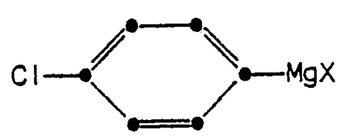

umgesetzt und α,α-Bis(4-chlorphenyl)-1H-imidazol-4(5)-methanol gebildet wird und das Produkt gegebenenfalls in ein Salz überführt wird.

3. Verfahren nach Anspruch 1, dadurch gekennzeichnet, daß Imidazol der Reihe nach mit einer starken Alkalimetallbase und einer Verbindung der Formel

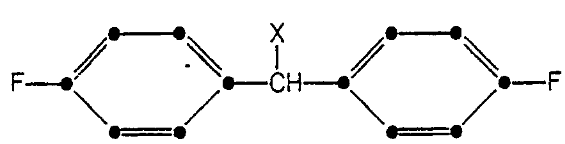

worin X Chlor oder Brom ist, umgesetzt und 4(5)-[Bis(4-fluorphenyl)methyl]-1H-imidazol gebildet wird und das Produkt gegebenenfalls in ein Salz überführt wird.

4. Verfahren nach Anspruch 1, dadurch gekennzeichnet, daß Imidazol der Reihe nach mit einer starken Alkalimetallbase und einer Verbindung der Formel

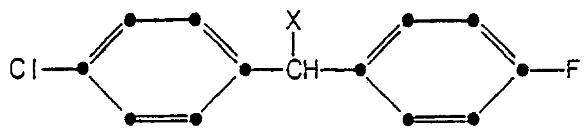

worin X Chlor oder Brom ist, umgesetzt und 4(5)-[(4-Chlorphenyl)(4-fluorphenyl)methyl]-imidazol gebildet wird und das Produkt gegebenenfalls in ein Salz überführt wird.

**Revendications pour les Etats contractants: BE CH LI DE FR GB IT LU NL SE**

1. Composé de formule I:

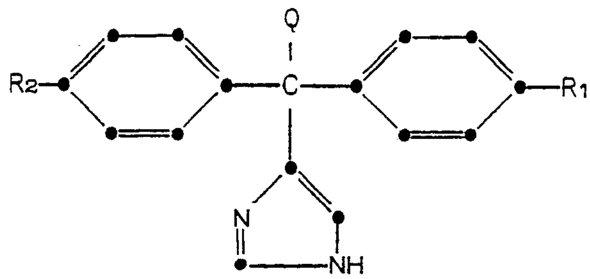

(I)

dans laquelle

Q représente un atome d'hydrogène ou un groupe hydroxyle, et

$R^1$ et $R^2$ représentent chacun indépendamment l'un de l'autre F ou Cl,

ou un de ses sels pharmaceutiquement acceptables.

2. α,α-bis-(4-chlorophényl)-1H-imidazole-4(5)-méthanol, ou un de ses sels pharmaceutiquement acceptables.

3. 4-[(4-chlorophényl)-(4-fluorophényl)méthyl]-1H-imidazole, ou un de ses sels pharmaceutiquement acceptables.

4. 4-bis-[(4-fluorophényl)méthyl]-1H-imidazole, ou un de ses sels pharmaceutiquement acceptables.

5. Utilisation d'un composé selon l'une quelconque des revendications 1 à 4 lors de la préparation d'un médicament en vue d'inhiber l'aromatase ou en vue de traiter ou de prévenir les maladies dépendant des oestrogènes.

6. Formulation pharmaceutique comprenant un composé selon l'une quelconque des revendications 1 à 4 comme ingrédient actif en association avec un ou plusieurs supports ou excipients pharmaceutiquement acceptables pour ce composé.

7. Procédé de préparation d'un composé de formule I:

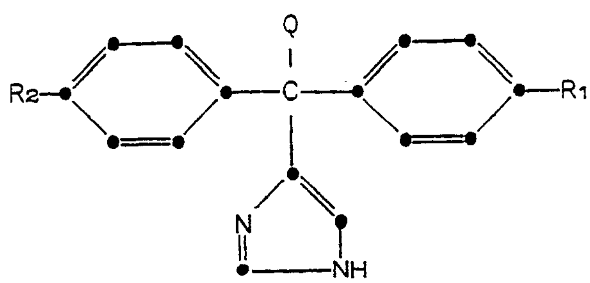

(I)

dans laquelle

Q représente un atome d'hydrogène ou un groupe hydroxyle, tandis que

$R^1$ et $R^2$ représentent chacun indépendamment l'un de l'autre F ou Cl,

ou d'un de ses sels pharmaceutiquement acceptables, caractérisé en ce qu'il comprend les étapes qui consistent à:

(a) faire réagir un composé de formule II:

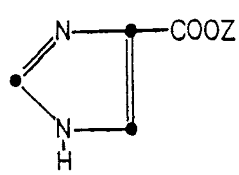

(II)

tout d'abord avec un réactif de Grignard de formule:

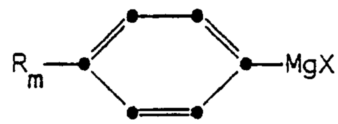

puis avec un réactif de Grignard de formule:

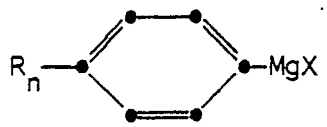

dans laquelle Z représente un groupe alkyle en $C_1$—$C_4$, un des radicaux $R_m$ et $R_n$ représente $R^1$, tandis que l'autre représente $R^2$ et X représente un atome de chlore ou un atome de brome, pour obtenir un composé de formule I dans laquelle Q représente un groupe hydroxyle, ou

(b) faire réagir un imidazole séquentiellement avec une base forte d'un métal alcalin et un composé de formule:

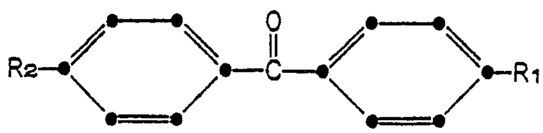

puis séparer les isomères obtenus pour former un composé de formule I dans laquelle Q représente un groupe hydroxyle, ou

(c) faire réagir un imidazole séquentiellement avec une base forte d'un métal alcalin et un composé de formule:

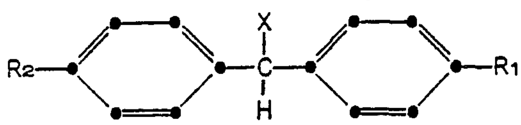

dans laquelle X représente un atome de chlore ou un atome de brome, puis séparer les isomères obtenus pour former un composé de formule I dans laquelle Q représente un atome d'hydrogène; et

(d) facultativement salifier le produit de formule I.

**Revendications pour l'Etat contractant: AT**

1. Procédé de préparation d'un composé de formule I:

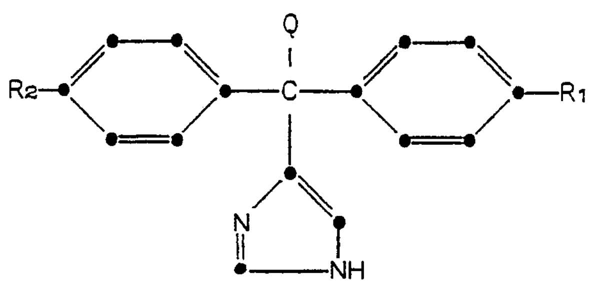

(I)

dans laquelle

Q représente un atome d'hydrogène ou un groupe hydroxyle, et

$R^1$ et $R^2$ représentent chacun indépendamment l'un de l'autre F ou Cl,

ou d'un de ses sels pharmaceutiquement acceptables, caractérisé en ce qu'il comprend les étapes qui consistent à:

(a) faire réagir un composé de formule II:

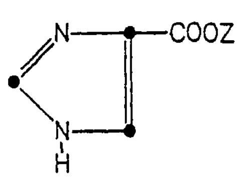

(II)

tout d'abord avec un réactif de Grignard de formule:

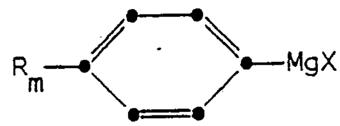

puis avec un réactif de Grignard de formule:

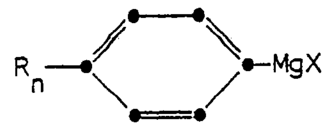

où Z représente un groupe alkyle en $C_1$—$C_4$, un des radicaux $R_m$ et $R_n$ représente $R^1$, tandis que l'autre représente $R^2$, et X représente un atome de chlore ou un atome de brome, pour obtenir un composé de formule I dans laquelle Q représente un groupe hydroxyle, ou

(b) faire réagir un imidazole séquentiellement avec une base forte d'un métal alcalin et un composé de formule:

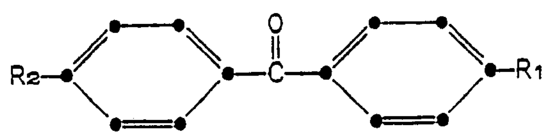

puis séparer les isomères obtenus pour former un composé de formule I dans laquelle Q représente un groupe hydroxyle, ou

(c) faire réagir un imidazole séquentiellement avec une base forte d'un métal alcalin et un composé de formule:

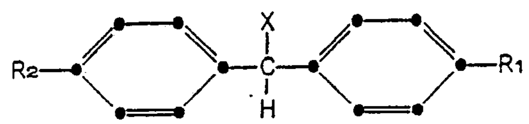

dans laquelle X représente un atome de chlore ou un atome de brome, puis séparer les isomères obtenus pour former un composé de formule I dans laquelle Q représente un atome d'hydrogène; et

(d) facultativement salifier le produit de formule I.

2. Procédé selon la revendication 1, dans lequel un composé de formule:

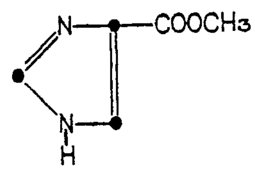

est mis à réagir avec au moins deux équivalents d'un réactif de Grignard de formule:

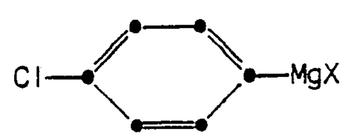

pour former l'α,α-bis-(4-chlorophényl)-1H-imidazole-4(5)-méthanol, tandis que le produit obtenu est facultativement salifié.

3. Procédé selon la revendication 1, dans lequel l'imidazole est mis à réagir séquentiellement avec une base forte d'un métal alcalin et un composé de formule:

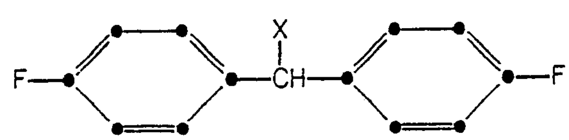

où X représente un atome de chlore ou un atome de brome, pour obtenir le 4(5)-[bis-(4-fluorophényl)méthyl]-1H-imidazole, le produit obtenu étant facultativement salifié.

4. Procédé selon la revendication 1, caractérisé en ce que l'imidazole est mis à réagir séquentiellement avec une base forte d'un métal alcalin et un composé de formule:

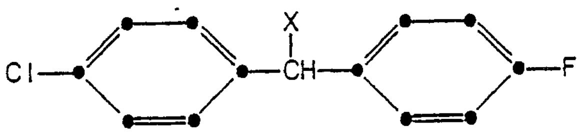

dans laquelle X représente un atome de chlore ou un atome de brome, pour obtenir le 4(5)-[(4-chlorophényl)-(4-fluorophényl)méthyl]-imidazole, le produit obtenu étant facultativement salifié.